# EUROPEAN PATENT SPECIFICATION

(11) **EP 3 752 984 B1**
(45) Date of publication and mention of the grant of the patent: **06.04.2022**
(21) Application number: 19703261.8
(22) Date of filing: 29.01.2019
(51) Int. Cl.: G06T 7/77, G06T 7/30, A61B 8/08

(54) **AN IMAGING SYSTEM AND METHOD WITH STITCHING OF MULTIPLE IMAGES**
BILDGEBENDES SYSTEM UND -VERFAHREN MIT STITCHING MEHRERER BILDER
SYSTÈME ET PROCÉDÉ D'IMAGERIE PAR PIQUAGE D'IMAGES MULTIPLES

(30) Priority: 14.02.2018 EP 18156749
(43) Date of publication of application: 23.12.2020
(73) Proprietor: Koninklijke Philips N.V., 5656 AG Eindhoven (NL)
(72) Inventor: SABCZYNSKI, Jörg, 5656 AE Eindhoven (NL); GRASS, Michael, 5656 AE Eindhoven (NL); PREVRHAL, Sven, Peter, 5656 AE Eindhoven (NL)
(74) Representative: Philips Intellectual Property & Standards
(86) International application number: PCT/EP2019/052029
(87) International publication number: WO 2019/158350

(56) References cited:
- SUN SHIH-YU ET AL: "Probe Localization for Freehand 3D Ultrasound by Tracking Skin Features", 14 September 2014 (2014-09-14), MEDICAL IMAGE COMPUTING AND COMPUTER-ASSISTED INTERVENTION - MICCAI 2015 : 18TH INTERNATIONAL CONFERENCE, MUNICH, GERMANY, OCTOBER 5-9, 2015; PROCEEDINGS; [LECTURE NOTES IN COMPUTER SCIENCE; LECT.NOTES COMPUTER], SPRINGER INTERNATIONAL PUBLISHING, CH, XP047318889, ISSN: 0302-9743 ISBN: 978-3-319-69952-3 abstract; figure 5 Section 2.2. "Enhanced Natural Skin Features"; 2.3 "Probe Localization in a Bayesian Probabilistic Framework"; pages 365-371
- BERGEN TOBIAS ET AL: "Stitching and Surface Reconstruction From Endoscopic Image Sequences: A Review of Applications and Methods", IEEE JOURNAL OF BIOMEDICAL AND HEALTH INFORMATICS, IEEE, PISCATAWAY, NJ, USA, vol. 20, no. 1, 1 January 2016 (2016-01-01), pages 304-321, XP011596591, ISSN: 2168-2194, DOI: 10.1109/JBHI.2014.2384134 [retrieved on 2015-12-31]
- MOZAFFARI MOHAMMAD HAMED ET AL: "Freehand 3-D Ultrasound Imaging: A Systematic Review", ULTRASOUND IN MEDICINE AND BIOLOGY, NEW YORK, NY, US, vol. 43, no. 10, 14 July 2017 (2017-07-14) , pages 2099-2124, XP085155762, ISSN: 0301-5629, DOI: 10.1016/J.ULTRASMEDBIO.2017.06.009
- NAGARAJ YESHASWINI ET AL: "Advanced inside-out tracking approach for real-time combination of MRI and US images in the radio-frequency shielded room using combination markers", 2016 38TH ANNUAL INTERNATIONAL CONFERENCE OF THE IEEE ENGINEERING IN MEDICINE AND BIOLOGY SOCIETY (EMBC), IEEE, 16 August 2016 (2016-08-16), pages 2558-2561, XP032979698, DOI: 10.1109/EMBC.2016.7591252
- YESHASWINI NAGARAJ ET AL: "US/MRI fusion with new optical tracking and marker approach for interventional procedures inside the MRI suite", CURRENT DIRECTIONS IN BIOMEDICAL ENGINEERING, vol. 2, no. 1, 30 September 2016 (2016-09-30), XP055476384, DOI: 10.1515/cdbme-2016-0101

## Description

### FIELD OF THE INVENTION

This invention relates to an imaging system and method in which multiple images of a structure of interest are stitched together.

### BACKGROUND OF THE INVENTION

Typically imaging techniques, such as ultrasound imaging, have a limited field of view. For some applications, e.g. the diagnosis of peripheral vascular or musculoskeletal disease, this field of view may be too small for an accurate and timely diagnosis.

To form larger images, known colloquially as panoramic images, a plurality of images from different positions and orientations of the ultrasonic probe are required. Panoramic imaging allows the display of complete structures and their relationship to surrounding anatomical features as well as quantitative long distance measurements. These images are presently stitched together by registration methods either solely based on imaging techniques and software, such as speckle correlation, or else based on external tracking devices which require a pre-calibrated set of cameras or additional hardware. In the case of imaging techniques, the registered images are prone to errors, are slow to produce, and have large distortions. A tracking approach is very costly as multiple high quality cameras positioned in specific locations and accompanying visual recognition software may be required. Other registration methods may involve optical measurement devices or electromagnetic tracking which are also costly and require additional hardware.

While usual optical position measurement devices offer high measurement speed and comparably high positional accuracy, their accuracy in measuring rotations may be limited, depending on the configuration of cameras and objects, and can be insufficient for the submillimeter accuracy required for some applications, such as panoramic ultrasound or single-photon emission computed tomography (SPECT) imaging.

A further problem is that the panoramic images produced do not have known positional relationship to specific features of the subject's anatomy, so that if further intervention is required, e.g. a needle puncture, it might be difficult to find the correct entry point on the subject's surface.

Sun Shih Yu et. al., "Probe localization for Freehand 3D Ultrasound by Tracking Skin Features" XP047318889 discloses a system for ultrasound probe localization which also involves skin surface mapping, for example using a camera mounted to the probe and a SLAM (simultaneous localization and mapping) algorithm.

### SUMMARY OF THE INVENTION

The invention is defined by the claims.

According to examples in accordance with an aspect of the invention, there is provided an imaging system, according to claim 1.

This imaging system generates stitched images of a structure of interest so that a large field of view may be achieved, thus forming a so-called panoramic image. By acquiring the image data and the images of a subject's surface and texture with a camera, preferably simultaneously, the orientation and positioning of the probe can be known without any additional hardware arrangements. In this way, image registration is unnecessary as the resultant imaged structure of interest is already formed from correctly aligned images.

The images of the surface provide landmarks on the subject's surface, and these may be visualized and used for interventional purposes. The accuracy with which locations on the surface may be identified is improved by providing a surface model of both shape and texture. By way of example, the surface model enables an operator to perform another scan at a later time (days, weeks or months later) while being able to locate the probe at the same location as previously, so that accurate comparisons can be made between scanned images.

The surface model enables tracking of the position and/or orientation of the probe. The image analysis also enables detection of when the probe has touched the subject.

The surface model is generated locally at the probe. The image stitching (of the image data, e.g. ultrasound image data) is preferably performed remotely. Because the surface model is created locally at the probe, only the surface model (shape and texture) in combination with coordinates need to be transmitted with the image data representing the probe position and orientation corresponding to that image data (e.g. ultrasound image). This enables the stitching to be performed with a minimum amount of data transmission from the probe.

The image stitching could also be performed at the probe in a fully integrated solution.

The stitching may be used to form a panoramic image formed from multiple 2D images, or it may be used to generate a 3D image of a volume from a set of 2D images.

In one embodiment the probe may be an ultrasound transducer. In another embodiment the probe may be a handheld single-photon emission computed tomography (SPECT) imaging device. In a further embodiment the probe may be a mobile X-ray unit.

The processor is preferably adapted to construct the surface model of the subject using a simultaneous localization and mapping (SLAM) algorithm.

With the use of a SLAM algorithm, such as those used in the robotic mapping and self-navigation industry, not only can the localization of the probe in space (i.e. on the subject's skin) be known, but also an additional image (i.e. a map of the subject's skin) can be created simultaneously.

The second camera enables the construction of the surface model using complementary information from the environment and the subject surface, thus increasing accuracy of the localization with two data sets rather than one. In particular, orientation information may be more accurate when using the second camera. The cameras and the probe have fixed and known positional and orientational relationships. The second camera may for example point to a ceiling or walls of the room in which the imaging is being performed. This provides an additional frame of reference.

Any camera which is employed in the system of the invention may be an optical, near-infrared, or hyperspectral camera. If there are no features in the camera image, such as vessels and skin folds, the construction of the surface model may not work optimally, as the localization function does not function well. Spectral ranges such as near-IR can be used as these show superficial vessels as well as more of the subject's surface detail. A combination of a range of spectra, known as hyperspectral imaging, can also be used to increase accuracy by way of acquiring more data about the environment as well as the subject's surface.

The camera signal is essentially a video stream which can be used by the surface model construction methods already known in the art to localize the camera in space (position and orientation) while simultaneously creating a map of the environment imaged by the camera (i.e. the subject's skin).

For examples in which the probe is an ultrasound transducer, since the ultrasound signal data and camera video data are coupled, preferably with the camera and ultrasound transducer rigidly coupled together, the orientation of the ultrasound image in space is also known if the transducer-camera unit is calibrated (i.e. the coordinate transform from camera space to ultrasound space is known).

A representation of the surface model is rendered on a display together with the image data (e.g. ultrasound). The representation for example comprises a 3D surface mesh of the subject's skin with representation of the skin surface texture and is rendered on a display with a spatial coupling to the image data.

The recorded images could be further segmented. For example, vascular flow maps may be generated and related to the subject's surface model when using an ultrasound probe in Doppler mode. Other simulations such as fractional flow reserve (FFR) and instantaneous wave-free ratio (iFR) may also be carried out on the segmented 3D images.

In the preferred embodiment the surface model is rendered on a display in spatial registration with a 3D image of the structure of interest.

The invention also provides a treatment system comprising:
an imaging system as defined above; and
an interventional treatment system for use in conjunction with the imaging system.

By way of example, if a treatment system makes use of an interventional X-ray system (C-arm) for example, an additional camera of the imaging system can be used to localize the image data and images of the surface relative to the X-ray system for multi-modal image integration.

The additional camera is preferably also fixed to the probe, but it points away from the surface of the subject to the interventional system (e.g. X-ray system). It could thus be used to localize the interventional system relative to the probe and after that localize the subject surface and the probe images relative to the interventional system.

According to examples in accordance with another aspect of the invention, there is provided an imaging method according to claim 8.

The construction of the surface model and the position tracking take place simultaneously as part of the image data processing.

This processing method does not rely on a series of image registrations between adjacent images, rather on the localization by the camera. Therefore, it creates volumetric images with long distance accuracy and low distortion.

The constructing of the surface model of the subject for example uses a simultaneous localization and mapping (SLAM) algorithm. Complementary information is obtained from the environment using a second camera.

The method comprises synchronously reading the signal from the camera and the signal from the probe for the creation of a map which comprises a 3D surface mesh of the surface.

The invention may be implemented at least in part in software.

These and other aspects of the invention will be apparent from and elucidated with reference to the embodiment(s) described hereinafter.

### BRIEF DESCRIPTION OF THE DRAWINGS

Examples of the invention will now be described in detail with reference to the accompanying drawings, in which:
Fig. 1 shows a probe on the surface of a subject with a camera for taking images for use in the surface model construction;
Fig. 2 shows an artistic representation of the multi-modal image data overlay for an Achilles tendon;
Fig. 3 shows a further probe with an additional camera for acquiring complementary images from the environment;
Fig. 4 shows an imaging method; and
Fig. 5 shows an ultrasound system which may form the ultrasound transducer part of the probe of Fig. 1.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an imaging system which combines a probe for obtaining image data in respect of a structure of interest below a surface of a subject, a first camera for obtaining images of the surface of the subject and a second camera for capturing images of the environment. A surface model of the subject's surface is obtained, and simultaneously the position and/or orientation of the probe is tracked on the surface. Image data acquired at different positons and orientations is stitched based on the tracked positon of the probe.

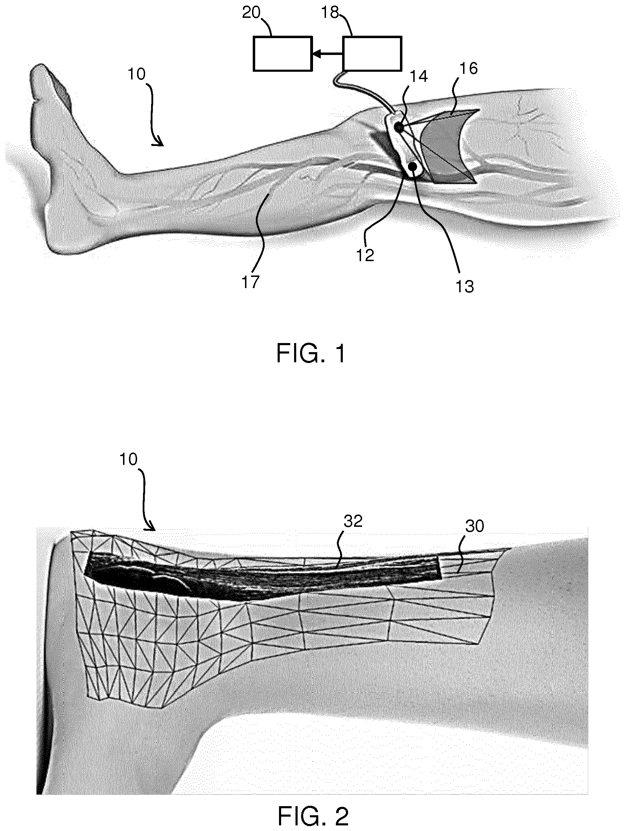

Fig. 1 shows a subject 10 with an ultrasound probe 12 on the subject's surface. The ultrasound probe 12 comprises an ultrasound head 13 for providing ultrasound imaging of a structure of interest beneath the surface of the subject. This imaging process creates image data. The term "image data" is thus used to represent data which results from the imaging of a volume of interest beneath the surface. Thus, in general it relies upon the use of an imaging signal which penetrates beneath the surface. As will be discussed further below, ultrasound imaging is only one example of a possible imaging modality.

In addition, the ultrasound probe 12 comprises a first camera 14 which is for example sensitive in the near-infrared spectrum, for collecting images of the surface. For producing surface images, there is no need for the imaging signal to penetrate beneath the surface, and visible light may be used. However, other wavelengths of light, such as the near-infrared spectrum, additionally allow some information to be obtained relating to the volume beneath the surface. The use of near-infrared sensing in particular allows the detection of superficial veins which may not be present with images from other spectra.

The image data provides information relating to the structure of interest to a greater depth than the images of the surface, even when these convey some depth-related information.

The area 16 within the field of view of the camera 14 is shown.

During the operation of the probe 12, the images of the surface collected by the camera 14 and the image data collected by the ultrasound head of the probe 12 are together provided to a processor 18 to construct a surface model. The surface model and the ultrasound image data are used to provide an output to a display device 20.

The processor 18 is part of the probe and is thus part of the apparatus which is scanned over the subject.

The surface model may be generated by a simultaneous localization and mapping (SLAM) algorithm, such as those used in the robotic mapping and self-navigation industry. SLAM is defined as the computational problem of constructing or updating a map of an unknown environment while simultaneously keeping track of the instrument's current location within the map. Several statistical methods may be used to solve this problem including, but not limited to, Kalman filters, particle filters (i.e. Monte Carlo methods) and scan matching of range data.

The SLAM algorithm can use the images from the camera 14 and the image data from the ultrasound head 13 to localize the probe 12 in space (i.e. on the subject's surface) while simultaneously creating a map (i.e. of the subject's skin). The superficial veins imaged by the near-infrared camera provide more detail for the SLAM algorithm than an optical image, for the mapping. As will be apparent to the skilled person in the art, a combination of various construction model techniques and image data sources such as near-infrared images, X-ray images, optical images etc., from the probe and camera may be used.

The image data in respect of different probe locations and/or orientations is stitched to form a 3D image of a 3D volume of interest. This stitched image is combined with the surface images to form a combined image in which the two image types are display in registration with each other, thereby forming a multi-modal image.

Fig. 2 shows an artistic representation of a multi-modal image for an Achilles tendon, which is then the structure of interest of the subject 10. The image comprises a 3D surface mesh 30 of the surface model in registration with the 3D stitched 3D ultrasound image 32. The 3D surface mesh 30 represents a map which has been created by the surface model algorithm during the use of the probe 12. The ultrasound image data is acquired simultaneously with the images of the surface. The position and orientation of the probe in space are known for each ultrasound image which allows the creation of the surface model. Registration based on processing of the surface images is then unnecessary due to the algorithm used and thus a real-time display with minimal distortion can be rendered on the display device 20. As the disclosed processing method does not rely on a series of registrations between adjacent images, but on the localization by the camera, it creates images with long distance accuracy.

As a technician operates probe 12, the SLAM algorithm can continually update the 3D surface mesh 30 as more data is acquired from the camera 14 which in turn can be used to update the 3D surface mesh 30. This continually updating surface could be shown on a display as it evolves in real-time or snapshots of the spatially coupled ultrasound image and surface mesh may be shown in predetermined intervals.

The images displayed in spatial coupling, may also be of use in simulations such as FFR/iFR which can be displayed on the display device 10. For such simulations, the images may need to be segmented - this can be performed by the processor 18.

Furthermore, vascular flow maps can be generated and related to the subject's surface model when using the ultrasound probe in Doppler mode. Although the surface mesh and the image data are coupled, the data from each may be individually stored and recalled later by some computer software (not shown). Thus functional imaging data (of which Doppler imaging is one example) may be added to the 3D data set.

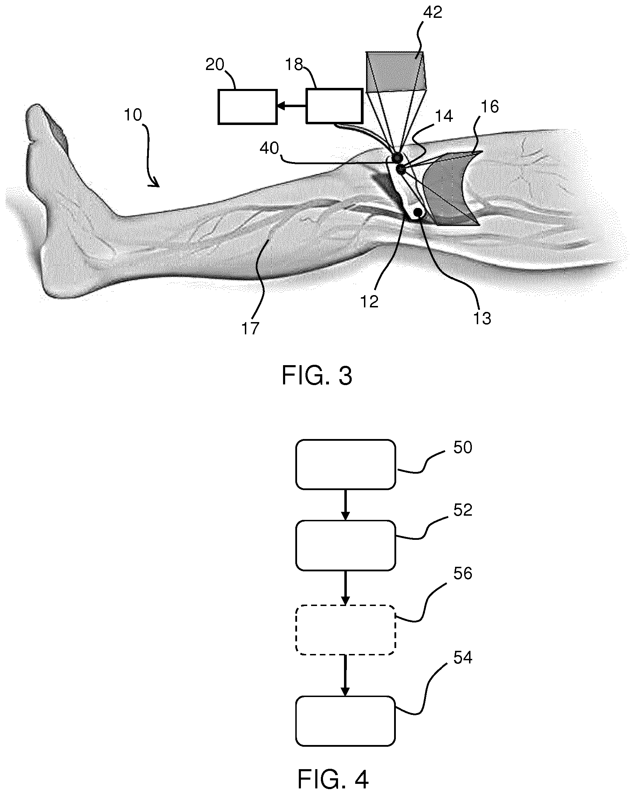

Fig. 3 shows a subject 10 with a probe 12 on their surface. Similar to Fig. 2, a first camera 14 is provided for taking surface images, which optionally include the superficial vessels 17, and example area 16 of these images is shown.

In accordance with the invention, Fig. 3 also depicts a second camera 40 present in the probe 12 (i.e. having a fixed positional relationship with respect to the probe, and in particular the field of view of the first camera is fixed relative to the field of view of the second camera, and they are each fixed relative to the orientation and position of the probe itself), and the area 42 of images that can be taken from this camera is also shown.

The purpose of second camera 40 is to obtain complimentary images from the surroundings; this may be the ceiling, another structure in the room or on the subject. This enables the construction of the surface model using complementary information from the surroundings and the subject surface, thus drastically increasing accuracy of the localization with two datasets rather than one.

The surroundings will include fixed features, and these can be identified by image analysis of the second camera images. Because the second camera is mounted at (e.g. on) the probe, orientation changes of the probe will result in movement of those fixed features in the captured images, and the relatively large distance to those fixed features effectively amplifies the effect of orientation changes, making the orientation estimation more accurate.

The second camera may also identify the location of other objects such as a other imaging systems or interventional treatment systems such as an X-ray arm. By mounting both cameras at the probe, there is no need for an external cameral system, for example to capture the location/orientation of the probe or the X-ray arm (or other equipment whose relative location is desired) in 3D space.

Often the goal of SLAM methods is to create a model of the environment. The identification of the camera position and orientation is just a necessary part of the processing carried out, but is not normally of separate value once the model of the environment has been created.

However, in the system of the invention, the position and orientation (pose) estimation is more important, because this information is used to stitch together the panoramic ultrasound image.

The camera is relatively close to the skin whereas the ultrasound image represents a structure much further away from the camera. This means that any inaccuracy in the position and orientation estimation, which is based on the camera image, will lead to a much higher inaccuracy of the stitching.

By arranging the second camera to point to a more remote scene, such as the ceiling which is much further away than the tissue surface, the accuracy of the orientation estimation is much higher as explained above.

Both cameras need to be calibrated, by which is meant their relative position and orientation to the probe needs to be known. Of course, this is a simple matter when they are mounted on, and therefore form a part of, the probe.

There are various ways to process the additional information obtained from the second camera.

A first approach is to implement position and orientation estimation independently on both camera image streams. Two results for each position and orientation would then be obtained. The first camera should give the better position, and the second (ceiling pointing) camera should give the better orientation estimation.

The position of the ultrasound image can be calculated from a weighted combination of the estimated position from the first camera and of the estimated position from the second camera. Similarly, the orientation of the ultrasound image can be calculated from a weighted combination of the estimated orientation from the first camera and of the estimated orientation from the second camera. The weights for the combination of the estimated results can take the different accuracies of the estimations into account. For example, the orientation estimation from the second camera may be more heavily weighted than from the first camera whereas the position estimation from the first camera may be more heavily weighted than from the second camera.

A second approach is to process streams and perform optimization with two surface maps, but just one position and orientation estimation. Thus, there is only one estimation for position and orientation, but with both image streams combined into two maps, one for each camera. However, when the first camera is pointing towards the patient and the second to the ceiling, there will be no overlap between the images from the first and second cameras, hence no overlap of the maps. In such a case, using two maps is essentially the same as using one map covering both fields of view. The algorithm for localizing and mapping would be the same in principle.

Thus, a third approach is to process image streams and perform optimization with an effective single surface map (representing both the surface of the subject and the surface away from the subject), and just perform one position and orientation estimation.

Note that the general term "a surface model" of the subject and of the environment is intended to include all these possibilities, i.e. two independent surface model portions which do not overlap, two independent surface model portions which do overlap, or a combined single surface model. Similarly tracking the position and/or orientation of the probe on the surface based on that surface model is intended to include obtaining a single position estimation and a single orientation estimation, or else combining two position estimations and/or two orientation estimations.

The second camera 40 and first camera 14 may be adapted to operate in a plurality of spectra or may be hyperspectral imaging devices; in any case, the cameras can be individually adapted to operate in different spectra pursuant to the respective structure the cameras will be imaging.

The images taken by the first camera 14 and the second camera 40 may be displayed on the display 20 in spatial registration with the ultrasound image 32 and the 3D surface mesh 30, or one set of images may be omitted and used purely for the enhancement in localization and orientation of the probe by the processor in the surface model construction, tracking and stitching.

The imaging system may be used as part of an overall treatment system which combines the imaging system with an interventional system. The imaging is then used to guide the interventional process, for example the location of an insertion to the body, or the direction of a laser or X-ray treatment.

By way of a first example, a needle insertion procedure may be performed by the following process:
The combined 3D ultrasound image dataset is created together with the surface mesh including surface texture;
A target lesion in the 3D image is identified automatically by computerized image analysis or else manually by a system operator. The computerized analysis then plans the entry point on the surface.

This planned entry point is then used as a manual map or else an overlay of the planned entry point may be provided on a live camera image which is registered with the combined dataset.

By way of a second example, quantitative follow-up imaging may be performed by the following process:
During a first examination, the combined 3D image dataset is created together with the surface mesh including texture;
During a second examination, the combined 3D image dataset is again created together with the surface mesh including texture.

The images are then matched based on surface feature registration and disease progression can then be determined accurately.

In this way, the generated surface model is used to find the position of a lesion in a later session, e.g. to compare time development of a lesion or to guide an invasive procedure.

Fig. 4 shows an imaging method that uses a probe and a camera. The method comprises:
in step 50, acquiring image data of a structure of interest of the subject beneath a surface of the subject with the probe;
in step 52, acquiring images of the surface with the camera; and
in step 54, processing the acquired image data from the probe and the acquired images from the camera to created stitched image data.

This processing comprises constructing a surface model of the surface based on the images of the surface (e.g. using a SLAM algorithm), tracking the position and/or orientation of the probe on the surface based on the constructed surface model and stitching the acquired image data based on the tracked position and/or orientation of the probe.

The method may comprise acquiring complementary information from the environment using a second camera, shown by step 56.

As described above, one application of the invention is for the stitching of ultrasound images from a hand-held ultrasound probe. The probe design may be entirely conventional, and the invention resides only in the generation of a stitched image by using surface mapping and probe tracking.

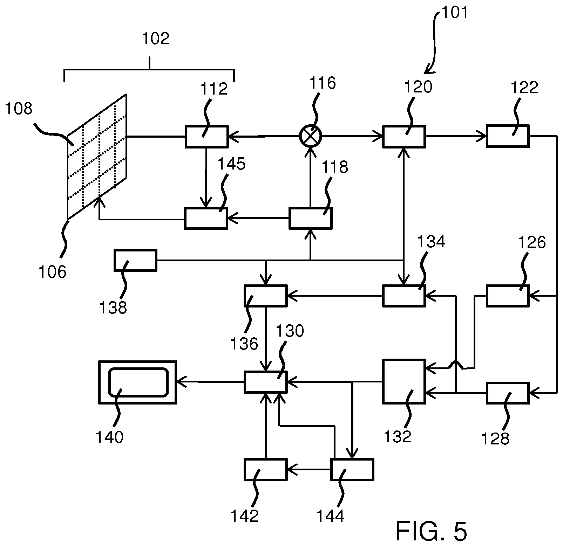

However, for completeness, a description will now be presented of the elements that make up a known ultrasound imaging system, in particular a diagnostic imaging system. The ultrasound transducer of the probe is thus part of a larger ultrasound diagnostic imaging system. Fig. 5 shows such an ultrasonic diagnostic imaging system 101 with an array transducer probe 102 in block diagram form.

The array transducer probe 102 comprises transducer cells. Traditionally, piezoelectric materials have been used for ultrasonic transducers. Examples are lead zirconate titanate (PZT) and polyvinylidene difluoride (PVDF), with PZT being particularly popular as the material of choice. The piezoelectric effect is a reversible process, meaning mechanically deformed piezoelectric crystals produce an internal electrical charge and also produce a mechanical strain when experiencing an applied electric field. The introduction of an alternating current (AC) to a piezoelectric material creates ultrasound pressure waves at a frequency related to the AC frequency. Single crystal piezoelectric materials are used to achieve high piezoelectric and electromechanical coupling constants for high-performance transducers.

Recent developments have led to the prospect that medical ultrasound transducers can be batch manufactured by semiconductor processes. Desirably, these processes should be the same ones used to produce the application specific integrated circuits (ASICs) needed by an ultrasound probe such as a complementary metal-oxide-semiconductor (CMOS) process, particularly for 3D ultrasound. These developments led to the production of micromachined ultrasonic transducers (MUTs), the preferred form being the capacitive MUT (CMUT). CMUT transducers are tiny diaphragm-like devices with electrodes that convert the sound vibration of a received ultrasound signal into a modulated capacitance.

CMUT transducers, in particular, can function over a broad bandwidth, enable high resolution and high sensitivity imaging, and produce a large pressure output so that a large depth of field of acoustic signals can be received at ultrasonic frequencies.

Fig. 5 shows a transducer array 106 of CMUT cells 108 as discussed above for transmitting ultrasonic waves and receiving echo information. The transducer array 106 of the system 101 may be a one- or a two-dimensional array of transducer elements capable of scanning in a 2D plane or in three dimensions for 3D imaging.

The transducer array 106 is coupled to a micro-beamformer 112 which controls transmission and reception of signals by the CMUT array cells. Beamforming is a method of signal processing that allows directional transmittance, or reception, of a signal such as ultrasound. Signals at particular angles undergo constructive or destructive interference in the transducer array 106 that allows desired signals to be selected and others ignored. Receive beamforming may also utilize a time delay for receiving signals due to the differences in echo depths.

Micro-beamformers are capable of at least partial beamforming by the application of delay-and-sum beamforming of the signals received of adjacent or small groups of transducer elements, for instance as described in US patents US 5,997,479 (Savord et al.), US 6,013,032 (Savord), and US 6,623,432 (Powers et al.). Micro-beamforming is often carried out inside the probe to reduce the number of signals sent to the main beamformer to be processed.

The micro-beamformer 112 is coupled by the probe cable, e.g., coaxial wire, to a transmit/receive (T/R) switch 116 which switches between transmission and reception modes and protects the main beam former 120 from high energy transmit signals when a micro-beamformer is not present or used. The transducer array 106 is operated directly by the main system beamformer 120. The transmission of ultrasonic beams from the transducer array 106 under control of the micro-beamformer 112 is directed by a transducer controller 118 coupled to the micro-beamformer by the T/R switch 116 and the main system beam former 120, which receives input from the user's operation of the user interface or control panel 138. One of the functions controlled by the transducer controller 118 is the direction in which beams are steered and focused. Beams may be steered straight ahead from (orthogonal to) the transducer array 106, or at different angles for a wider field of view possibly by delaying excitation pulses sent from the array transducer cells.

The transducer controller 118 may be coupled to control a voltage source 145 for the transducer array. For instance, the voltage source 145 sets DC and AC bias voltage(s) that are applied to the CMUT cells of a CMUT array 106, e.g., to generate the ultrasonic RF pulses in transmission mode.

The partially beam-formed signals produced by the micro-beamformer 112 are forwarded to the main beamformer 120 where partially beam-formed signals from individual patches of transducer elements are combined into a fully beam-formed signal. For example, the main beam former 120 may have 128 channels, each of which receives a partially beam-formed signal from a patch of dozens or hundreds of CMUT transducer cells 108. In this way, the signals received by thousands of transducer elements of a transducer array 106 can contribute efficiently to a single beam-formed signal.

The beam-formed signals are coupled to a signal processor 122. The signal processor 122 can process the received echo signals in various ways, such as bandpass filtering, decimation, I and Q component separation, the demodulation of a wave and its sample 90 degrees out of phase and harmonic signal separation, which acts to separate linear and nonlinear signals so as to enable the identification of nonlinear (higher harmonics of the fundamental frequency) echo signals returned from tissue and microbubbles.

The signal processor 122 optionally may perform additional signal enhancement such as speckle reduction, signal compounding and noise elimination. The bandpass filter in the signal processor 122 may be a tracking filter, with its passband sliding from a higher frequency band to a lower frequency band as echo signals are received from increasing depths, thereby rejecting the noise at higher frequencies from greater depths where these frequencies are devoid of anatomical information.

The processed signals are coupled to a Bright-mode (B-mode) processor 126 and optionally to a Doppler processor 128. The B-mode processor 126 employs detection of amplitude of the received ultrasound signal for the imaging of structures in the body, such as the tissue of organs and vessels.

B-mode images of the structure of the body may be formed in the harmonic image mode or in the fundamental image mode or a combination of both as described in US Patent 6,283,919 (Roundhill et al.) and US Patent 6,458,083 (Jago et al.).

The Doppler processor 128, if present, processes temporally distinct signals from tissue movement and blood flow for the detection of the motion of substances, such as the flow of blood cells in the image field. The Doppler processor typically includes a wall filter with parameters which may be set to pass and/or reject echoes returned from selected types of materials in the body. For instance, the wall filter can be set to have a passband characteristic which passes signal of relatively low amplitude from higher velocity materials while rejecting relatively strong signals from lower or zero velocity material.

This passband characteristic will pass signals from flowing blood while rejecting signals from nearby stationary or slowing moving objects such as the wall of the heart. An inverse characteristic would pass signals from moving tissue of the heart while rejecting blood flow signals for what is referred to as tissue Doppler imaging, detecting and depicting the motion of tissue. The Doppler processor receives and processes a sequence of temporally discrete echo signals from different points in an image field; the sequence of echoes from a particular point referred to as an ensemble. An ensemble of echoes received in rapid succession over a relatively short interval can be used to estimate the Doppler shift frequency of flowing blood, with the correspondence of the Doppler frequency to velocity indicating the blood flow velocity. An ensemble of echoes received over a longer period of time is used to estimate the velocity of slower flowing blood or slowly moving tissue.

The structural and motion signals produced by the B-mode (and Doppler) processor(s) are coupled to a scan converter 132 and a multiplanar reformatter 144. The scan converter 132 arranges the echo signals in the spatial relationship from which they were received in the desired image format. For instance, the scan converter may arrange the echo signal into a two dimensional (2D) sector-shaped format, or a pyramidal three dimensional (3D) image.

The scan converter can overlay a B-mode structural image with colors corresponding to motion at points in the image field with their Doppler-estimated velocities to produce a color Doppler image which depicts the motion of tissue and blood flow in the image field. The multiplanar reformatter 144 will convert echoes which are received from points in a common plane in a volumetric region of the body into an ultrasonic image of that plane, as described further in US Patent 6,443,896 (Detmer). The minimum amount of data points required to describe a plane is three, one can then move in a direction orthogonal to the plane some fixed amount after measuring those three points and repeat that plane measurement, thus building a volumetric region without acquiring data from the entire volume itself. A volume renderer 142 converts the echo signals of a 3D data set into a projected 3D image as viewed from a given reference point as described in US Patent 6,530,885 (Entrekin et al.)

The 2D or 3D images are coupled from the scan converter 132, multiplanar reformatter 144, and volume renderer 142 to an image processor 130 for further enhancement, buffering and temporary storage for display on an image display 140. In addition to being used for imaging, the blood flow values produced by the Doppler processor 128 and tissue structure information produced by the B-mode processor 126 are coupled to a quantification processor 134. The quantification processor produces measures of different flow conditions such as the volume rate of blood flow as well as structural measurements such as the sizes of organs and gestational age, for example. The quantification processor may receive input from the user control panel 138, such as the point in the anatomy of an image where a measurement is to be made.

Output data from the quantification processor is coupled to a graphics processor 136 for the reproduction of measurement graphics and values with the image on the display 140. The graphics processor 136 can also generate graphic overlays for display with the ultrasound images. These graphic overlays can contain standard identifying information such as patient name, date and time of the image, imaging parameters, and the like. For these purposes, the graphics processor receives input from the user interface 138, such as patient name.

The user interface 138 is also coupled to the transmit controller 118 to control the generation of ultrasound signals from the transducer array 106 and hence the images produced by the transducer array 106 and the ultrasound system 101. The user interface 138 is also coupled to the multiplanar reformatter 144 for selection and control of the planes of multiple multiplanar reformatted (MPR) images which may be used to perform quantified measures in the image field of the MPR images.

As will be understood by the skilled person, the above embodiment of an ultrasonic diagnostic imaging system is intended to give a non-limiting example of such an ultrasonic diagnostic imaging system. The skilled person will immediately realize that several variations in the architecture of the ultrasonic diagnostic imaging system are feasible. For instance, as also indicated in the above embodiment, the micro-beamformer 112 and/or the Doppler processor 128 may be omitted, the ultrasound probe 102 may not have 3D imaging capabilities and so on. Other variations will be apparent to the skilled person.

In preferred implementations, a 3D image of a 3D volume beneath the surface is obtained at each probe position and orientation. The stitched image is then a 3D image of a large volume than covered by each individual probe position and orientation. However, the image data to be stitched may comprise 2D images, such as images of a slice of a 3D volume, and these 2D images are stitched to create a 3D image of the 3D volume.

As explained above, the processor 18 is part of the probe 12. The construction of the surface model is locally at the probe. The probe then derives the position and orientation information of the probe and provides this information with the image data and the surface model including the shape and texture.

In one approach, the ultrasound image stitching is performed at the back end processor. However, the ultrasound image stitching may instead be performed at the probe, so that the panoramic image is output from the probe.

It will also be understood that the present invention is not limited to an ultrasonic diagnostic imaging system. The teachings of the present invention are equally applicable to a variation whereby the probe is an X-ray imaging unit, a single-photon emission computed tomography (SPECT) imaging unit, or some other investigative imaging. As will be immediately apparent to the skilled person, in such other imaging systems the system components described with reference Fig. 4 will be adapted accordingly.

The invention makes use of a camera which can image surface texture. This is not possible with a structured light source, which instead maps only surface contour.

Thus, the system makes use of a non-structured light source to enable surface texture to be visible. This surface texture for example comprises skin folds, hairs, follicles or nevi. "Surface texture" is also intended to include surface coloration. Thus "surface texture" may be understood to mean localized surface shape, features and color.

The system may however additional include a structured light source approach, for example with alternating use of a structured light source for surface contour mapping and a non-structured light source for surface texture mapping.

As mentioned above, the surface model is generated at the probe. The other processing may be performed at the probe or remotely on a computer to which the probe is attached. An ultrasound scanner with the form factor of a mobile phone with a display included could for example be used to perform all of the processing.

One example of possible division of processing task is as follows:
The camera images are collected at the probe;
The surface mapping using a SLAM algorithm is carried out at the probe;
Ultrasound images are sent from the probe to backend, and the probe location and orientation for each ultrasound image is attached to the image;
The probe also sends the surface map to the backend;
The backend stitches the ultrasound images;
The backend combines the surface map with the stitched panoramic ultrasound image;
The backend sends the combined images to the display.

SLAM algorithms are known for surface mapping. However, other surface mapping algorithms may be used.

The stitching process used may be conventional. In particular, given the position and orientation from which images are captured by a particular camera, it is well known how to stitch such images together to form a panoramic view. In particular, image reconstructions as well as volume reconstructions from multiple images are well known.

For example, endoscopic image stitching methods are discussed in Bergen Tobias et. al., "Stitching and Surface Reconstruction from Endoscopic Image Sequences: A Review of Applications and Methods" XP011596591.

Other variations to the disclosed embodiments can be understood and effected by those skilled in the art in practicing the claimed invention, from a study of the drawings and the disclosure, without departing from the scope of the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. An imaging system, comprising:
a probe (12) adapted to be placed on or near the surface of a subject to acquire image data of a structure of interest of the subject beneath the surface,
wherein the probe comprises:
an imaging transducer for obtaining the image data;
a first camera (14) adapted to acquire images of the surface;
a second camera (40) adapted to acquire complementary information from the environment, the second camera being arranged to point to a remote scene of the environment comprising one or more fixed features, the distance between the second camera and the remote scene being greater than the distance between the first camera and the surface, wherein the second camera is adapted to identify a movement of the one or more fixed features in the captured images of the remote scene as the position and/or orientation of the probe changes, wherein the imaging transducer, the first camera and second camera have fixed positional and orientational relationship with respect to the probe; and
a processor (18), adapted to synchronously process the acquired image data from the imaging transducer and the acquired images from the first and second cameras to construct a surface model of (i) the shape and texture of the surface of the subject based on the images of the surface and (ii) the environment based on the second camera complementary information, and track the position and/or orientation of the probe (12) on the surface based on the constructed surface model of the shape and texture of the surface of the subject and of the environment; and
a processor adapted to stitch the image data acquired at different positions and/or orientations based on the tracked position and/or orientation of the probe.

2. An imaging system as claimed in claim 1, wherein the probe comprises, as the imaging transducer:
an ultrasound transducer (13); or
a single-photon emission computed tomography (SPECT) imaging device; or
an X-ray unit.

3. An imaging system as claimed in claim 1 or 2, wherein:
the image data comprises 2D images and the stitched image data comprises a 2D image with a larger field of view; or
the image data comprises 2D images and the stitched image data comprises a 3D image of a 3D volume.

4. An imaging system as claimed in any preceding claim, wherein the processor (18) of the probe is adapted to construct the surface model of the subject using a simultaneous localization and mapping (SLAM) algorithm.

5. An imaging system as claimed in any preceding claim, wherein the first and/or second camera (14, 40) comprises an optical, near-infrared, or hyperspectral camera.

6. An imaging system as claimed in any proceeding claim, comprising a display device (20), wherein the processor (18) is adapted to represent the images of the surface of the subject and the image data in registration using the display device (20).

7. A system comprising:
an imaging system as claimed in any preceding claim; and
an additional system for performing an interventional procedure, wherein the imaging system is adapted to guide the additional system during the interventional procedure.

8. An imaging method that uses a probe which comprises an imaging transducer and first and second cameras, wherein the imaging transducer, the first camera and second camera have fixed positional and orientational relationship with respect to the probe, the method comprising:
(50) acquiring image data of a structure of interest of the subject beneath a surface of the subject using the imaging transducer of the probe with the probe placed on or near the surface of the subject;
(52) acquiring images of the surface with the first camera;
(56) using the second camera to acquire complementary information from the environment, the second camera being arranged to point to a remote scene of the environment comprising one or more fixed features, the distance between the second camera and the remote scene being greater than the distance between the first camera and the surface, wherein the second camera is adapted to identify a movement of the one or more fixed features in the captured images of the remote scene as the position and/or orientation of the probe changes; and
in the probe, (54) synchronously processing the acquired image data from the probe and the acquired images from the first and second cameras, the processing comprising constructing a surface model of (i) the shape and texture of the surface of the subject based on the images of the surface and (ii) the environment based on the second camera complementary information, and tracking the position and/or orientation of the probe on the surface based on the constructed surface model of the shape and texture of the surface of the subject and of the environment; and
stitching the the image data acquired at different positions and/or orientations based on the tracked position and/or orientation of the probe.

9. A method as claimed in claim 8, wherein the constructing of the surface model of the subject uses a simultaneous localization and mapping (SLAM) algorithm.

10. A method as claimed in one of claims 8 to 9, comprising displaying the signal from the first camera and the signal from the probe simultaneously using the display device.

11. A computer program comprising instructions which, when the program is executed by a computer, cause the computer to carry out the method of any one of claims 8 to 10.

## Patentansprüche

1. Ein Bildgebungssystem, umfassend:
eine Sonde (12), angepasst, um auf oder in der Nähe einer Oberfläche eines Gegenstands platziert zu werden und Daten einer Struktur des Gegenstandes zu erfassen, der von Interesse ist, wobei die Sonde umfasst:
einen Umformer für die Bildgebung, um die Bilddaten zu erhalten; eine erste Kamera (14), angepasst, um Bilder von der Oberfläche zu erhalten; eine zweite Kamera (40), angepasst, um ergänzende Informationen aus der Umgebung zu erfassen, wobei die zweite Kamera angeordnet ist, um auf eine entfernte Szene der Umgebung zu zeigen, die ein oder mehrere feste Merkmale umfasst, wobei der Abstand zwischen der zweiten Kamera und der entfernten Szene größer ist als der Abstand zwischen der ersten Kamera und der Oberfläche. Die zweite Kamera ist angepasst, um eine Bewegung des einen oder der mehreren festen Merkmale in den erfassten Bildern der entfernten Szene zu identifizieren, wenn sich die Position und/oder Ausrichtung der Sonde ändert, wobei der Umformer für Bildgebung, die erste Kamera und die zweite Kamera eine feste Position und Orientierung in Bezug auf die Sonde aufweisen; und ein Prozessor (18), angepasst, um in synchroner Weise die Bilddaten vom Umformer und die erhaltenen Bilder von der ersten und zweiten Kamera zu verarbeiten und damit ein Oberflächenmodell (i) der Form und Textur der Oberfläche des Gegenstands auf Grundlage der Bilder der Oberfläche und (ii) der Umgebung auf Grundlage der zusätzlichen Informationen der zweiten Kamera zu konstruieren und die Position und/oder Orientierung der Sonde (12) auf der Oberfläche aufgrund des konstruierten Oberflächenmodells der Form und Textur der Oberfläche des Gegenstands und der Umgebung nachzuverfolgen; und einen Prozessor, angepasst, um die Bilddaten zusammenzufügen, die an den verschiedenen Positionen und/oder Orientierungen auf Grundlage der nachverfolgten Position und/oder Orientierung der Sonde erhalten wurden.

2. Ein Bildgebungssystem wie in Anspruch 1 beansprucht, wobei die Sonde als Bildgebungs-Umwandler: einen Ultraschall-Umwandler (13) umfasst; oder ein Bildgebungsgerät auf Basis Computergestützter Einzelphotonen-Tomografie (SPECT); oder ein Röntgengerät.

3. Ein Bildgebungssystem wie in Anspruch 1 oder 2, wobei: die Bilddaten 2D-Bilder umfassen, und die zusammengefügten Bilddaten enthalten ein 2D-Bild mit einem größeren Sichtfeld; oder die Bilddaten 2D-Bilder umfassen, und die zusammengefügten Bilddaten enthalten ein 3D-Bild mit einem 3D-Umfang.

4. Ein Bildgebungssystem wie in einem der vorherigen Ansprüche beansprucht, wobei der Prozessor (18) der Sonde angepasst ist, das Oberflächenmodell des Gegenstands unter Verwendung eines Algorithmus zu gleichzeitiger Lokalisierung und Mapping (SLAM) zu konstruieren.

5. Ein Bildgebungssystem wie in einem der vorherigen Ansprüche, wobei die erste und/oder zweite Kamera (14, 40) eine optische, Nah-Infrarot- oder hyperspektrale Kamera umfassen.

6. Ein Bildgebungssystem wie in einem der vorherigen Ansprüche beansprucht, welches ein Display-Gerät (20) umfasst, wobei der Prozessor (18) angepasst ist, die Bilder der Oberfläche des Gegenstands und die Bilddaten in Registrierung unter Verwendung des Display-Gerätes (20) abzubilden.

7. Ein System, welches: ein Bildgebungssystem wie in einem der vorherigen Ansprüche umfasst; und ein zusätzliches System zur Durchführung eines Eingriffs, wobei das Bildgebungssystem angepasst ist, das zusätzliche System während des Eingriffs zu leiten.

8. Eine Bildgebungsmethode, die eine Sonde verwendet, welche einen Bildgebungs- Umwandler und eine erste und zweite Kamera umfasst, wobei der Bildgebungs-Umwandler und die erste und zweite Kamera feste Positions- und Orientierungsbeziehungen im Verhältnis zur Sonde haben. Die Methode umfasst:
(50) das Erfassen von Bilddaten einer Struktur von Interesse des Gegenstands unter der Oberfläche des Gegenstands unter Verwendung des Bildgebungs-Umwandlers der Sonde, wobei die Sonde auf der Oberfläche des Gegenstands oder in der Nähe davon platziert ist; (52) das Erfassen von Bildern der Oberfläche mit der ersten Kamera; (56) Verwenden der zweiten Kamera, um komplementäre Informationen aus der Umgebung zu erfassen, wobei die zweite Kamera angeordnet ist, um auf eine entfernte Szene der Umgebung zu zeigen, die ein oder mehrere feste Merkmale umfasst, wobei der Abstand zwischen der zweiten Kamera und der entfernten Szene größer ist als der Abstand zwischen der ersten Kamera und der Oberfläche, wobei die zweite Kamera angepasst ist, um eine Bewegung des einen oder die mehreren festen Merkmale in den erfassten Bildern der entfernten Szene zu identifizieren in dem Maß, wie die Position und/oder Ausrichtung der Sonde sich ändert; und in der Sonde, (54) wobei die in synchroner Weise die Bilddaten vom Umformer und die erhaltenen Bilder von der ersten und zweiten Kamera verarbeitet werden. Die Verarbeitung umfasst den Aufbau eines Oberflächenmodells (i) der Form und Textur der Oberfläche des Gegenstands auf Grundlage der Bilder der Oberfläche und (ii) der Umgebung auf Grundlage der zusätzlichen Informationen der zweiten Kamera, und erlaubt es, die Position und/oder Orientierung der Sonde auf der Oberfläche aufgrund des konstruierten Oberflächenmodells der Form und Textur der Oberfläche des Gegenstands und seiner Umgebung nachzuverfolgen; und es werden die Bilddaten zusammengefügt, die an den verschiedenen Positionen und/oder Orientierungen auf Grundlage der nachverfolgten Position und/oder Orientierung der Sonde erhalten wurden.

9. Eine Methode wie in Anspruch 8 beansprucht, wobei der Aufbau des Oberflächenmodells des Gegenstands einen Algorithmus zu gleichzeitiger Lokalisierung und Mapping (SLAM) verwendet.

10. Eine Methode wie in Anspruch 8 bis 9 beansprucht, welche eine Darstellung des Signals der ersten Kamera und des Signals der Sonde gleichzeitig unter Verwendung des Display-Geräts umfasst.

11. Ein Computerprogramm, welches Anweisungen umfasst, die, wenn das Programm durch einen Computer ausgeführt wird, den Computer veranlassen, eine der Methoden eines der Ansprüche 8 bis 10 auszuführen.

## Revendications

1. Un système d'imagerie, comprend : une sonde (12) adaptée pour être placée sur ou près d'une surface d'un objet pour acquérir des données d'imagerie d'une structure d'intérêt du sujet sous la surface, où la sonde comprend: un transducteur d'imagerie pour obtenir des données d'imagerie; une première caméra (14) adaptée pour obtenir des images de la surface; une deuxième caméra (40) adaptée pour obtenir des informations complémentaires de l'environnement, la seconde caméra étant disposée pour pointer vers une scène distante de l'environnement comprenant une ou plusieurs caractéristiques fixes, la distance entre la seconde caméra et la scène distante étant plus grande que la distance entre la première caméra et la surface, où la seconde caméra est adaptée pour identifier un mouvement d'une ou plusieurs caractéristiques fixes dans les images saisies de la scène distante lorsque la position ou l'orientation de la sonde change, où le transducteur d'imagerie, la première caméra et la seconde caméra ont une relation fixe de position et d'orientation par rapport à la sonde; et un processeur (18), adapté de manière synchronisée les données d'imagerie obtenue depuis le transducteur d'imagerie, et les images obtenues depuis la première et seconde caméra pour construire un modèle de surface de (i) la forme et la texture de la surface du sujet fondée à partir d'images de la surface et (ii) l'environnement fondé sur l'information complémentaire de la seconde caméra, et suivre la position ou l'orientation de la sonde (12) sur la surface fondée sur le modèle de la surface construite de la forme et de la texture de la surface du sujet et de l'environnement; et un processeur adapté pour assembler les données d'imagerie acquises dans différentes positions ou orientations fondées à partir de la position suivie ou l'orientation de la sonde.

2. Un système d'imagerie comme revendiqué dans la revendication 1, où la sonde comprend, un transducteur d'imagerie: un transducteur à ultrasons (13) ; ou une tomographie par émission mono photonique (SPECT) un appareil d'imagerie; ou une unité de rayons X.

3. Un système d'imagerie comme revendiqué dans la revendication 1 ou 2, où : des données d'imagerie comprennent des images 2D et les images assemblées de données comprennent une image 2D avec un champ de vision plus large; ou des données d'imagerie comprennent des images 2D et les images assemblées de données comprennent une image 3D d'un volume 3D.

4. Un système d'imagerie comme revendiqué selon l'une quelconques des revendications précédentes, où le processeur (18) de la sonde est adapté pour construire le modèle de surface du sujet en utilisant une localisation simultanée et un algorithme de cartographie (SLAM).

5. Un système d'imagerie comme revendiqué selon l'une quelconques des revendications précédentes, où la première ou seconde caméra (14, 40) comprend une caméra optique, proche infrarouge ou hyperspectrale.

6. Un système d'imagerie comme revendiqué selon l'une quelconques des revendications précédentes comprend un dispositif d'affichage (20), où le processeur (18) est adapté pour représenter les images d'une surface du sujet et les données d'imagerie enregistrées en utilisant le dispositif d'affichage (20) .

7. Un système d'imagerie comprend: un système d'imagerie comme revendiqué selon l'une quelconques des revendications précédentes; et un système additionnel pour la réalisation d'une procédure interventionnelle, où le système d'imagerie est adapté pour guider le système additionnel pendant la procédure interventionnelle.

8. Une méthode d'imagerie qui utilise une sonde qui comprend un transducteur d'imagerie et une première et seconde caméra, où le transducteur d'imagerie, la première et seconde caméra ont une relation fixe de position et d'orientation par rapport à la sonde, la méthode comprenant: (50) l'obtention d'imagerie de données d'une structure d'intérêt du sujet sous une surface du sujet utilisant le transducteur d'imagerie de la sonde avec la sonde placée sur ou près d'une surface du sujet; (52) l'obtention d'images de la surface avec la première caméra; (56) l'utilisation de la seconde caméra pour obtenir des informations complémentaires depuis l'environnement, la seconde caméra étant disposée de manière à pointer vers une scène distante de l'environnement comprenant une ou plusieurs caractéristiques fixes, la distance entre la seconde caméra et la scène distante étant plus grande que la distance entre la première caméra et la surface, où la seconde caméra est adaptée pour identifier un mouvement d'une ou plusieurs caractéristiques fixes dans les images saisies de la scène distante lorsque la position ou l'orientation de la sonde change; et dans la sonde (54) traiter de manière synchronisée les données d'imagerie obtenues depuis la sonde et les données obtenues depuis la première et seconde caméra, le traitement comprenant la construction d'un modèle de surface de (i) la forme et la texture de la surface du sujet fondée à partir d'images de la surface et (ii) de l'environnement fondé à partir de l'information complémentaire de la seconde caméra, et le suivi de la position ou l'orientation de la sonde sur la surface fondée à partir du modèle de la surface construite de la forme et de la texture de la surface du sujet et de l'environnement; et de l'assemblage des données d'imagerie obtenues dans différentes positions ou orientations fondées à partir du suivi de la position ou de l'orientation de la sonde.

9. Une méthode comme revendiquée dans la revendication 8, où la construction d'un modèle de surface du sujet utilise une localisation simultanée et un algorithme de cartographie (SLAM).

10. Une méthode comme revendiquée selon l'une des revendications 8 à 9, comprenant un affichage du signal depuis la première caméra et le signal depuis la sonde en utilisant simultanément le dispositif d'affichage.

11. Un programme informatique comprenant des instructions qui, lorsque le programme est exécuté par un ordinateur, font en sorte que l'ordinateur exécute la méthode selon l'une quelconque des revendications 8 à 10.
